# EUROPEAN PATENT APPLICATION

(11) **EP 4 495 107 A1**
(43) Date of publication of application: **22.01.2025**
(21) Application number: 22931736.7
(22) Date of filing: 23.09.2022
(51) Int. Cl.: C07D 307/24, C07D 405/06, C07D 319/06, C07C 67/11, C07C 51/31, C07C 45/41, C07C 69/732, C07C 59/56, C07C 49/235

(54) **PREPARATION METHOD FOR INTERMEDIATE COMPOUND ACTING AS SYNTHETIC POSACONAZOLE, AND INTERMEDIATE COMPOUND PREPARED THEREBY**

(30) Priority: 18.03.2022 CN 202210274526
(71) Applicant: Zhejiang Ausun Pharmaceutical Co., Ltd., Taizhou, Zhejiang 317016 (CN)
(72) Inventor: DAI, Chunguang, Zhejiang 317016 (CN); ZHU, Baoyun, Zhejiang 317016 (CN); ZHANG, Lirong, Zhejiang 317016 (CN); PU, Xuecan, Zhejiang 317016 (CN); YU, Guanneng, Zhejiang 317016 (CN); LI, Yunfeng, Zhejiang 317016 (CN); ZHENG, Zhiguo, Zhejiang 317016 (CN)
(74) Representative: Carpmaels & Ransford LLP
(86) International application number: PCT/CN2022/120774
(87) International publication number: WO 2023/173717

(57) **Abstract**

The present disclosure relates to a method for preparing a compound as an intermediate for synthesizing the drug posaconazole and the intermediate compounds prepared thereby. The method for preparing a compound of Formula I provided in the present disclosure comprises: firstly reacting 4-(2,4-difluorophenyl)pent-4-enoate or its reduction product 4-(2,4-difluorophenyl)pent-4-enal as a starting material with formaldehyde or paraformaldehyde in the presence of a chiral catalyst, and then subjecting the reaction product to a characteristic oxidation, an esterification, a halogenation and cyclization reaction, finally a hydrolysis and an acid treatment. The preparation method of the present disclosure has advantages such as mild reaction conditions, simple reaction process, and high overall yield and high purity of target products, and thus is suitable for industrial production.

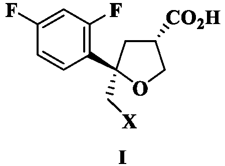

## Description

### Technical Field

The present disclosure relates to the field of pharmaceutical chemistry, and particularly to a method for preparing an intermediate compound as an intermediate for posaconazole and the intermediate compounds prepared by the method.

### Background

As a second generation antifungal drug, posaconazole is used for patients with low immunity, patients for whom the use of other antifungal agents such as amphothericin B, fluconazole, and itraconazole is ineffective, or patients who are intolerant to these antifungal agents. Posaconazole first came into the market in Europe on October 25, 2005, and then was approved by U.S. FDA on September 15, 2006. The compound of Formula IX below is a key intermediate for synthesizing the drug posaconazole (hereinafter referred to as "key intermediate for posaconazole"), which may be further converted into posaconazole through the following reaction scheme:

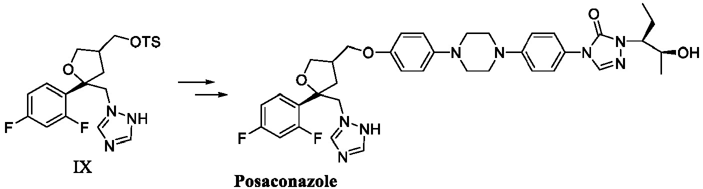

Currently, there are some literatures reporting methods for preparing the compound of Formula IX. For example, U.S. Patent Application No. US5661151A provides a method for preparing the main ring of posaconazole (Scheme 1). In this method, 2'-chloro-2,4-difluoroacetophenone as a starting material is subjected to a Witting reaction, resulting in inversion of configuration to finally obtain the compound of Formula IX. 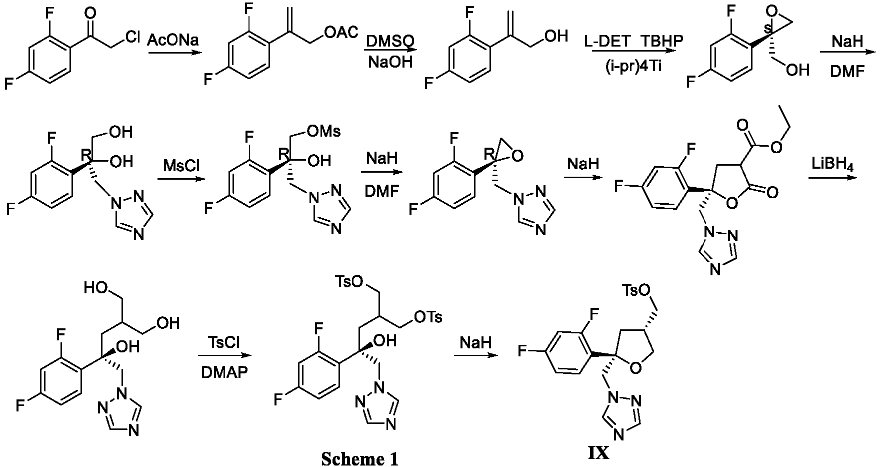 However, this reaction scheme has lengthy reaction steps, the dangerous strong base of NaH is used in at least four steps, and the too dangerous peroxide of tert-butyl hydroperoxide (TBHP) is further used in the third step.

In the CN patent publication No. CN103635465A of Sandoz AG (Switzerland) published in 2012, a reaction route wherein 2'-chloro-2,4-difluoroacetophenone is used as a starting material, and the Witting reaction is replaced by preparing a Grignard reagent is disclosed (Scheme 2). 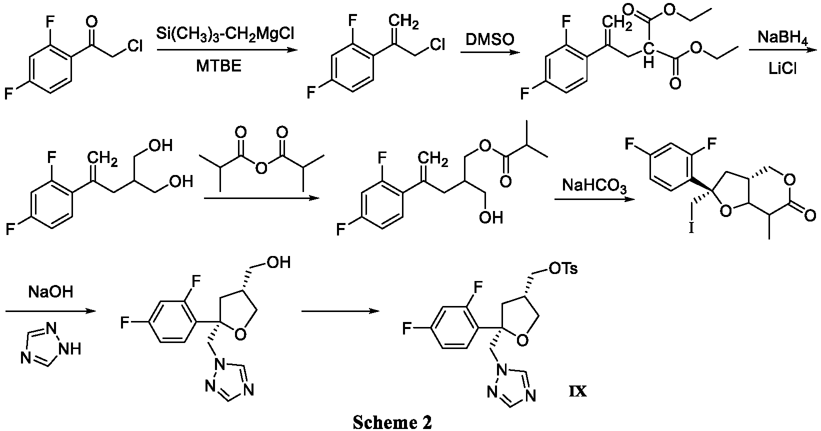 Although the reaction steps of this reaction scheme have been slightly shortened, and the reaction conditions are relatively mild, the final product has a higher content of isomers, which needs to be purified twice or more, resulting in relatively large loss.

The CN patent publication No. CN101824009A discloses a reaction route wherein 1-chloro-2,4-difluoroacetophenone is subjected to a Witting reaction to obtain 2-(2,4-difluorophenyl)propenol, which is then subjected to multiple reaction steps such as Sharpless epoxidation, nucleophilic substitution, condensation, and reduction to obtain the product (Scheme 3). 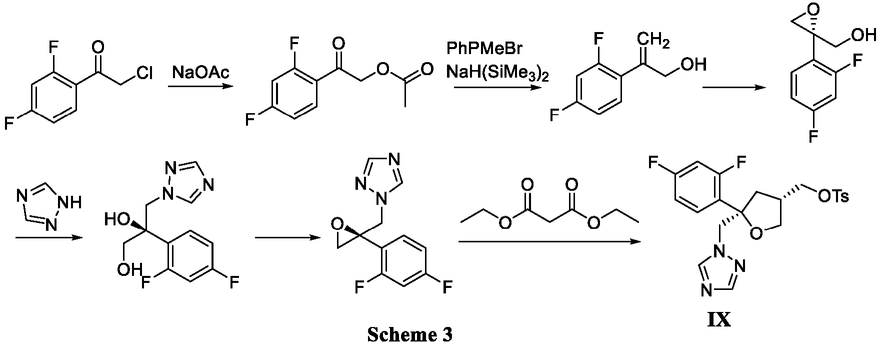 However, in the multiple reaction steps of this reaction scheme, column chromatography is used for separation and purification of the intermediates, resulting in low yield and high production cost, which is unfavorable for industrial production.

In 2012, MSN Laboratories Limited (India) discloses a synthetic route in US2014343285A1, in which m-difluorobenzene as a starting material is subjected to Friedel-Crafts acylation, a Witting reaction, condensation amidation, hydroxymethylation, halogenation and cyclization, reduction, aminoalkylation, and esterification to obtain the target product (Scheme 4). 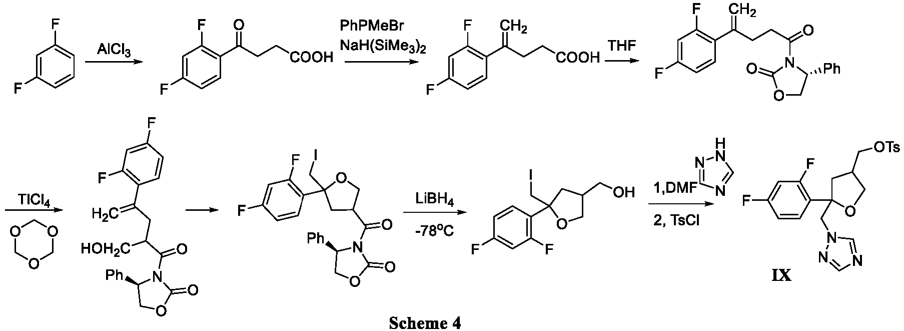 However, this reaction scheme is prone to produce intramolecular ring-closed impurities, which can be removed but affect the yield. Also, in this reaction process, flammable and expensive n-butyl lithium and hygroscopic and corrosive titanium tetrachloride are used for preparing 2-oxazolidinone lithium salt, so that it is not suitable for large-scale production.

Therefore, there is a need in the art for a method for preparing a compound as an intermediate for synthesizing posaconazole (hereinafter referred to as "intermediate for posaconazole"), especially a new method for preparing a compound of Formula IX as a key intermediate for posaconazole.

### Summary

In view of this, an object of the present disclosure is to overcome the above shortcomings and provide a method for preparing a compound as an intermediate for posaconazole, especially a new method for preparing a compound of Formula IX as a key intermediate for posaconazole. These methods have advantages such as mild reaction conditions, simple reaction process, high overall yield and high purity of target products, and are particularly suitable for industrial production. The present disclosure provides a new method for synthesizing a compound of Formula I, (*3S,5R*)-5-(halomethyl)-5-(2,4-difluorophenyl)tetrahydrofuran-3-carboxylic acid.

To this end, in one aspect, the present disclosure provides a method for preparing a compound of Formula I as an intermediate for posaconazole, 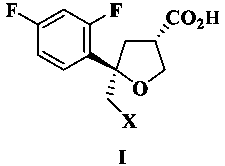 the method comprises the following step (1): hydrolyzing a compound of Formula II in a mixed solvent of an organic solvent and water under an alkaline condition, and then subjecting the hydrolysate to an acid treatment to obtain the compound of Formula I, 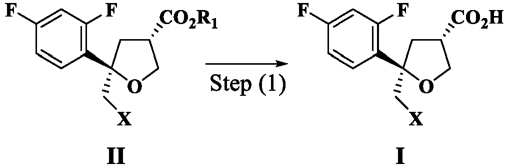 wherein X is Cl, Br or I, and R₁ is an ester protection group.

In some preferred embodiments, R₁ is selected from the group consisting of C₁₋₈ alkyl, C₃₋₈ cycloalkyl, C₆₋₁₀ aryl, C₁₋₈ alkyl-substituted C₆₋₁₀ aryl, benzyl, C₁₋₈ alkyl-substituted benzyl, C₁₋₈ alkoxy-substituted benzyl and halogenated benzyl; preferably, R₁ is selected from the group consisting of C₁₋₈ alkyl, benzyl, C₁₋₈ alkyl-substituted benzyl, C₁₋₈ alkoxy-substituted benzyl and halogenated benzyl.

In some preferred embodiments, the hydrolyzing in the step (1) is carried out in the presence of one or more bases selected from the group consisting of lithium hydroxide, sodium hydroxide, potassium hydroxide, sodium carbonate, potassium carbonate, sodium bicarbonate and potassium bicarbonate.

In some preferred embodiments, the acid treatment comprises adjusting the pH of an aqueous phase obtained after the reaction to 2~3 with one or more organic acids or inorganic acids; preferably, an organic amine is added to the solution obtained after the acid treatment to obtain an amine salt of the compound of Formula I.

In some preferred embodiments, the compound of Formula II is prepared by a method comprising the following steps (2) to (5):
step (2): reacting a compound of Formula VI with formaldehyde or paraformaldehyde in an organic solvent in the presence of a chiral catalyst to obtain a compound of Formula V;
step (3): subjecting the compound of Formula V to a Pinnick oxidation using NaClO₂ and NaH₂PO₄ in the presence of a scavenger to obtain a compound of Formula IV;
step (4): reacting the compound of Formula IV with a halohydrocarbon R₁-X under an alkaline condition to obtain a compound of Formula III; and
step (5): subjecting the compound of Formula III to a halogenation and cyclization reaction in the presence of a halogenated reagent containing halogen X to obtain the compound of Formula II,
   or
step (2): reacting a compound of Formula VI with formaldehyde or paraformaldehyde in an organic solvent in the presence of a chiral catalyst to obtain a compound of Formula V;
step (3): subjecting the compound of Formula V to a Pinnick oxidation using NaClO₂ and NaH₂PO₄ in the presence of a scavenger, and thereto adding an organic amine compound to obtain an organic amine salt of the compound of Formula IV;
step (4): reacting the organic amine salt of the compound of Formula IV with a halohydrocarbon R₁-X under an alkaline condition to obtain a compound of Formula III; and
step (5): subjecting the compound of Formula III to a halogenation and cyclization reaction in the presence of a halogenated reagent containing halogen X to obtain the compound of Formula II, 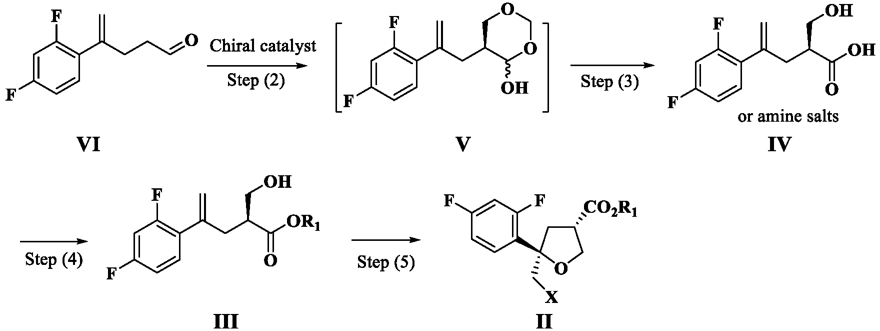 wherein X and R₁ are as defined above.

In some preferred embodiments, the chiral catalyst used in the step (2) is a compound of the following formula: 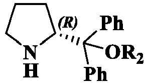 wherein R₂ is selected from the group consisting of trimethylsilyl, triethylsilyl, *tert*-butyldimethylsilyl, triisopropylsilyl, benzyl, 4-methylbenzyl and phenyl, and Ph represents phenyl.

In some preferred embodiments, the Pinnick oxidation is performed in an acetonitrile-water solution in the presence of NaClO₂ and NaH₂PO₄; preferably, the scavenger is one or more selected from the group consisting of resorcinol, aminosulfonic acid, 2-methyl-2-butene, dimethyl sulfoxide and hydrogen peroxide.

In some preferred embodiments, the halogenated reagent containing halogen X used in the step (5) is one or more selected from the group consisting of halogen, dichlorohydantoin, dibromohydantoin, diiodohydantoin, *N*-chlorosuccinimide (NCS), *N*-bromosuccinimide (NBS) and *N*-iodosuccinimide (NIS); preferably, the organic amine is one or more selected from the group consisting of methylamine, ethylamine, propylamine, cyclopropylamine, *n*-butylamine, *tert*-butylamine, *n*-pentylamine, isopentylamine, *tert*-pentylamine, cyclopentylamine, hexylamine, cyclohexylamine, diethylamine, ethylenediamine, diisopropylethylamine, triethylamine, ethanolamine, phenylamine, phenylethylamine and benzylamine.

In some preferred embodiments, the compound of Formula VI is prepared via reduction of a compound of Formula X:

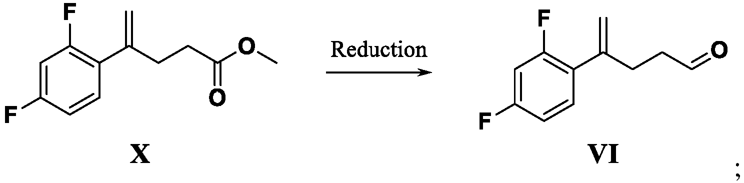

Preferably, the used reducing agent is one or more selected from the group consisting of lithium borohydride, sodium borohydride, potassium borohydride, sodium cyanoborohydride, potassium cyanoborohydride, borane, lithium aluminum hydride, diisobutylaluminum hydride and Red-Al.

In another aspect, the present disclosure provides a method for preparing a compound of Formula IX as a key intermediate for posaconazole from a compound of Formula II, comprising the following step (1) and steps (6) to (8):
step (1): hydrolyzing a compound of Formula II in a mixed solvent of an organic solvent and water under an alkaline condition, and then subjecting the hydrolysate to an acid treatment to obtain the compound of Formula I;
step (6): reducing a compound of Formula I in an organic solvent in the presence of a reducing agent at a temperature in a range of -5~5°C to obtain a compound of Formula VII;
step (7): reacting the compound of Formula VII with 1,2,4-triazole in an organic solvent in the presence of a base and a phase transfer catalyst to obtain a compound of Formula VIII; and
step (8): reacting the compound of Formula VIII with *p*-toluenesulfonyl chloride in an organic solvent under an alkaline condition to obtain the compound of Formula IX, 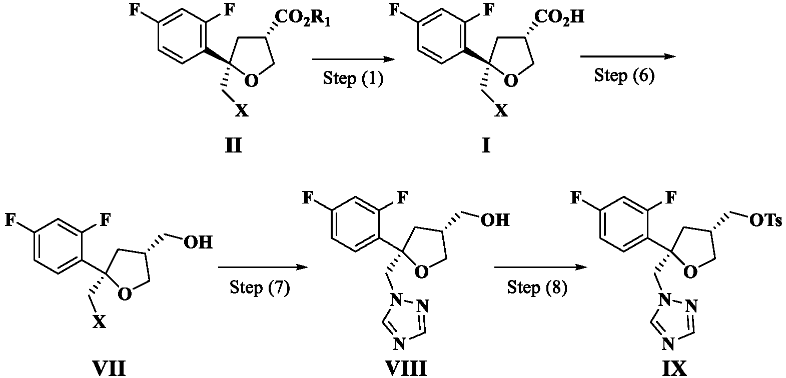 wherein X is Cl, Br or I; R₁ is an ester protection group; and Ts represents p-toluenesulfonyl.

In some preferred embodiments, the reducing agent used in the step (6) is one or more selected from the group consisting of lithium borohydride, sodium borohydride, potassium borohydride, sodium cyanoborohydride, potassium cyanoborohydride, borane, lithium aluminum hydride, diisobutylaluminum hydride and Red-Al; preferably, the reducing agent is a combination of one or more selected from the group consisting of lithium borohydride, sodium borohydride, potassium borohydride, sodium cyanoborohydride, potassium cyanoborohydride, borane, lithium aluminum hydride, diisobutylaluminum hydride and Red-Al with one or more selected from the group consisting of boron trifluoride diethyl etherate, aluminum chloride, ferric chloride and iodine.

In some preferred embodiments, the compound of Formula II is prepared by the method as described above.

In yet another aspect, the present disclosure provides a compound of Formula II prepared by the method as described above: 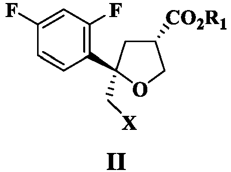 wherein X and R₁ are as defined above.

In yet another aspect, the present disclosure provides compounds of Formula II, Formula III, Formula IV, Formula V and Formula VI prepared by the method as described above: 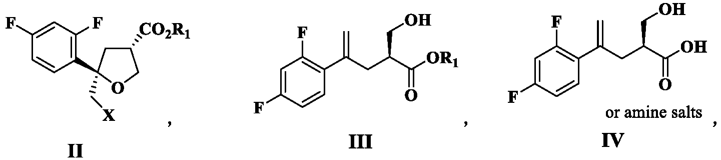 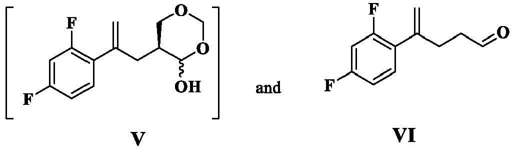 wherein R₁ is benzyl, and X is Cl, Br or I.

The present disclosure provides a new method for preparing a compound as an intermediate for posaconazole, especially a new method for preparing a compound of Formula IX as a key intermediate for posaconazole. These preparation methods have advantages such as mild reaction conditions, simple reaction process, and high overall yield and high purity of target products, which thus are suitable for industrial production.

In particular, the present disclosure uses a chiral catalyst to carry out the cyclization, the prepared compound of Formula IX has an enantiomeric excess (ee) value up to 99.5%, meaning a very high chiral purity.

Furthermore, the method for preparing a compound of Formula I from a compound of Formula VI provided in the present disclosure has an overall yield up to about 45%, and the method for preparing a compound of Formula IX from a compound of Formula I also has a relatively high overall yield up to about 66.0%.

Furthermore, the compound of Formula IX as a key intermediate for posaconazole prepared by the method of the present disclosure has a low impurity content and a high purity. Also, other intermediate compounds prepared by the method of the present disclosure have a low impurity content and a relatively high purity.

Furthermore, raw and auxiliary materials used in the method of the present disclosure are easily available and inexpensive, the reaction conditions are mild, and the operations are simple and convenient, such that large-scale production can be easily realized.

### Detailed Description

In view of one or more, or even all of the problems such as a high impurity content, a low purity, low yield, harsh reaction conditions, and being unfavorable for industrial production, the present inventors have extensively studied and found a new method for preparing a compound as an intermediate for posaconazole, especially a new method for preparing a compound of Formula IX as a key intermediate for posaconazole. These preparation methods have advantages such as mild reaction conditions, a simple reaction process, and a high overall yield and a high purity of target products, which thus are suitable for industrial production.

Based on this, the present disclosure provides a method for preparing a compound of Formula I as an intermediate for posaconazole. 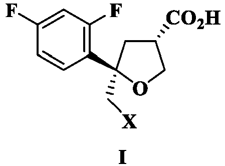 The method comprises the following step (1): hydrolyzing a compound of Formula II in a mixed solvent of an organic solvent and water under an alkaline condition, and then subjecting the hydrolysate to an acid treatment to obtain the compound of Formula I, 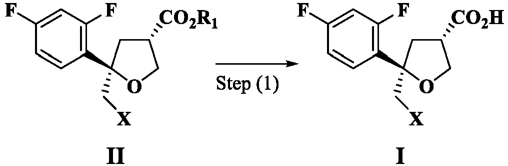 wherein X is Cl, Br or I, and R₁ is an ester protection group.

In the present disclosure, any ester protection group known in the art may be used. Preferably, as the ester protection group, R₁ may be selected from the group consisting of C₁₋₈ alkyl, C₃₋₈ cycloalkyl, C₆₋₁₀ aryl, C₁₋₈ alkyl-substituted C₆₋₁₀ aryl, benzyl (Bn), C₁₋₈ alkyl-substituted benzyl, C₁₋₈ alkoxy-substituted benzyl and halogenated benzyl; more preferably, R₁ is selected from the group consisting of C₁₋₈ alkyl, benzyl, C₁₋₈ alkyl-substituted benzyl, C₁₋₈ alkoxy-substituted benzyl and halogenated benzyl.

As used herein, C₁₋₈ alkyl refers to a linear or branched saturated hydrocarbyl containing 1 to 8 carbon atoms. Examples of C₁₋₈ alkyl includes, but not limited to, methyl, ethyl, propyl, isopropyl, n-butyl, isobutyl, *tert-butyl,* n-pentyl, isopentyl, n-hexyl, n-heptyl, n-octyl and the like. Accordingly, C₁₋₈ alkoxy refers to a group of C₁₋₈ alkyl-O-.

As used herein, C₃₋₈ cycloalkyl refers to a cyclic saturated hydrocarbyl containing 3 to 8 ring carbon atoms. Examples of C₃₋₈ cycloalkyl includes, but not limited to, cyclopropyl, cyclobutyl, cyclopentyl, cyclohexyl, cycloheptyl, cyclooctyl, methylcyclobutyl and the like.

As used herein, C₆₋₁₀ aryl refers to an aryl group containing 6 to 10 ring atoms. Examples of C₆₋₁₀ aryl includes, but not limited to, phenyl, naphthyl and the like. Accordingly, C₁₋₈ alkyl-substituted C₆₋₁₀ aryl refers to a group in which one or more hydrogen atoms on the aromatic ring carbon atoms of the C₆₋₁₀ aryl are substituted by one or more C₁₋₈ alkyl groups.

As used herein, benzyl refers to a group of C₆H₅-CH₂-. Accordingly, C₁₋₈ alkyl-substituted benzyl refers to a group in which one or more hydrogen atoms on the phenyl ring of the benzyl are substituted by one or more C₁₋₈ alkyl groups, and C₁₋₈ alkoxy-substituted benzyl or halogenated benzyl refers to a group in which one or more hydrogen atoms on the phenyl ring of the benzyl are substituted by one or more C₁₋₈ alkoxy groups.

In the above method, the hydrolysis in the step (1) may be carried out under any alkaline condition known in the art. Preferably, the hydrolysis in the step (1) is carried out in the presence of one or more bases selected from the group consisting of lithium hydroxide, sodium hydroxide, potassium hydroxide, sodium carbonate, potassium carbonate, sodium bicarbonate and potassium bicarbonate.

In the above method, the acid treatment may be carried out by using one or more organic acids or inorganic acids. Preferably, the acid treatment may comprise adjusting the pH of an aqueous phase obtained after the reaction to 2~3.

More specifically, in the above method, the compound of Formula II may be first dissolved in an organic solvent, an aqueous solution of one or more bases as described above may be added thereto, and the temperature may be controlled for example at -10~20°C for reaction. After the reaction is completed, impurities are removed by extraction with an organic solvent, and an acid is added to the aqueous phase to adjust the pH to 2~3. Then, the organic phase is extracted with an organic solvent, and then is concentrated to obtain the compound of Formula I as an oil.

Alternatively or optionally, after the acid is added to the aqueous phase to adjust the pH to 2~3, an organic solvent may be added to extract the compound of Formula I, and then an organic amine may be added to the organic phase to form an amine salt of the compound of Formula I, which is precipitated in the form of solid. Finally, the organic amine salt of the compound of Formula I may be obtained through filtration. Through the method of forming an organic amine salt, on one hand, some impurities may be effectively removed so as to increase the purity of the compound of Formula I; on the other hand, the amine salt is precipitated in the form of solid, facilitating the product isolation, such that the industrial production is more operative and the production cost is lower.

In the above method, the organic solvent used is not particularly limited, as long as it can dissolve the starting material compounds. Preferably, the organic solvent used may be one or more selected from the group consisting of toluene, hexane, *n*-heptane, methyl acetate, ethyl acetate, isopropyl acetate, acetonitrile, tetrahydrofuran, isopropyl ether, methyl *tert-butyl* ether, dichloromethane, acetone, methanol, ethanol, and isopropanol.

In the above method, the water used as a solvent is not particularly limited, which is used mainly for dissolving the base in the reaction, for example, to form an aqueous base solution. Preferably, drinking water, deionized water, distilled water and the like may be used.

In the above method, the acid used is not particularly limited, which may be any one or more organic acids or inorganic acids. Preferably, the acid used may be one or more selected from the group consisting of hydrochloric acid, hydrobromic acid, hydroiodic acid, sulfuric acid, nitric acid, phosphoric acid, formic acid and trifluoroacetic acid.

In the above method, the organic amine used is not particularly limited. Preferably, the organic amine used may be one or more selected from the group consisting of methylamine, ethylamine, propylamine, cyclopropylamine, *n*-butylamine, *tert*-butylamine, *n*-pentylamine, isopentylamine, tert-pentylamine, cyclopentylamine, hexylamine, cyclohexylamine, diethylamine, ethylenediamine, diisopropylethylamine, triethylamine, ethanolamine, phenylamine, phenylethylamine and benzylamine.

In the above method, preferably, the compound of Formula II is prepared by a method comprising the following steps (2) to (5):
step (2): reacting a compound of Formula VI with formaldehyde or paraformaldehyde in an organic solvent in the presence of a chiral catalyst to obtain a compound of Formula V;
step (3): subjecting the compound of Formula V to a Pinnick oxidation using NaClO₂ and NaH₂PO₄ in the presence of a scavenger to obtain a compound of Formula IV;
step (4): reacting the compound of Formula IV with a halohydrocarbon R₁-X under an alkaline condition to obtain a compound of Formula III; and
step (5): subjecting the compound of Formula III to a halogenation and cyclization reaction in the presence of a halogenated reagent containing halogen X to obtain the compound of Formula II,
   or
step (2): reacting a compound of Formula VI with formaldehyde or paraformaldehyde in an organic solvent in the presence of a chiral catalyst to obtain a compound of Formula V;
step (3): subjecting the compound of Formula V to a Pinnick oxidation using NaClO₂ and NaH₂PO₄ in the presence of a scavenger, and thereto adding an organic amine compound to obtain an organic amine salt of the compound of Formula IV;
step (4): reacting the organic amine salt of the compound of Formula IV with a halohydrocarbon R₁-X under an alkaline condition to obtain a compound of Formula III; and
step (5): subjecting the compound of Formula III to a halogenation and cyclization reaction in the presence of a halogenated reagent containing halogen X to obtain the compound of Formula II, 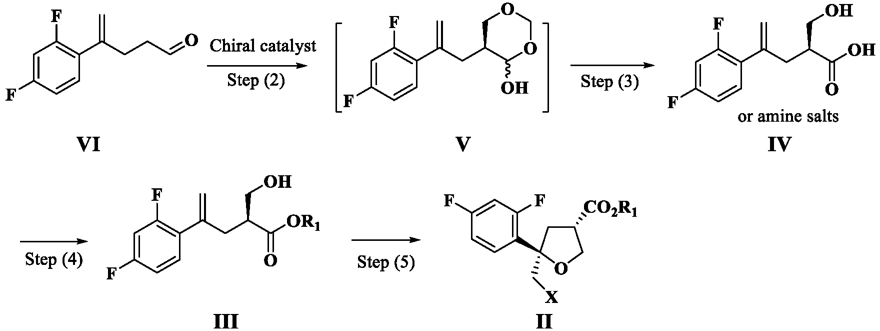 wherein X and R₁ are as defined above.

More specifically, in the step (2), the compound of Formula VI may be reacted with formaldehyde or paraformaldehyde, in a solvent such as toluene, chlorobenzene, ethyl acetate, isopropyl acetate, hexane, *n*-heptane, isopropyl ether or methyl *tert-butyl* ether, in the presence of the chiral catalyst and in the presence of hydroxide, carbonate, bicarbonate, phosphate or hydrogen phosphate of an alkaline metal or an alkaline earth metal, to obtain the compound of Formula V.

In the step (3), the compound of Formula V is reacted in a NaClO₂/NaH₂PO₄/acetonitrile-H₂O solution in the presence of the scavenger at a temperature not higher than 30°C, and then the reaction product is subjected to post-treatment to obtain the compound of Formula IV as an oil. Alternatively or optionally, in the step (3), the compound of Formula V is oxidized in the presence of NaClO₂/NaH₂PO₄, and thereto an organic amine is added to obtain an organic amine salt of the compound of Formula IV, which is then precipitated in the form of solid, such that the compound of Formula IV is effectively isolated from the solvent, and the purity is increased.

In the step (4), the compound of Formula IV obtained in the step (3) is reacted with the halogenated reagent containing halogen X under an alkaline condition at a temperature in a range of 5~20°C while maintaining the temperature. After the reaction is completed, the reaction product is subjected to post-treatment to obtain the compound of Formula III. Alternatively or optionally, the organic amine salt of the compound of Formula IV obtained in the step (3) is subjected to an acid treatment to release free compound of Formula IV, which is then reacted with the halogenated reagent containing halogen X under an alkaline condition at a temperature in a range of 5~20°C while maintaining the temperature. After the reaction is completed, the reaction product is subjected to post-treatment to obtain the compound of Formula III.

In the above method, preferably, the chiral catalyst used in the step (2) may be a compound of the following formula: 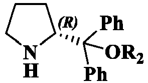 wherein R₂ may be selected from the group consisting of trimethylsilyl, triethylsilyl, tert-butyldimethylsilyl, triisopropylsilyl, benzyl, 4-methylbenzyl and phenyl, and Ph represents phenyl.

In the above method, the chiral catalyst used may be prepared, for example, according to the method described in the literature of Bioorganic and Medicinal Chemistry, 2017, 5(18), 4996-5001. Preferably, (*R*)-diphenylprolinol is reacted with R₂-X, and the reaction product is subjected to post-treatment to obtain the chiral catalyst used in the above method.

In the above method, preferably, the hydroxide, carbonate, bicarbonate, phosphate or hydrogen phosphate of an alkaline metal or an alkaline earth metal used in the step (2) may be one or more selected from the group consisting of lithium hydroxide, sodium hydroxide, potassium hydroxide, calcium hydroxide, sodium carbonate, potassium carbonate, cesium carbonate, sodium bicarbonate, potassium bicarbonate, sodium phosphate, potassium phosphate, sodium dihydrogen phosphate, potassium dihydrogen phosphate, calcium phosphate, calcium hydrogen phosphate, disodium hydrogen phosphate and dipotassium hydrogen phosphate. More preferably, the base used in the step (2) is selected from the combination of sodium dihydrogen phosphate-disodium hydrogen phosphate or the combination of potassium dihydrogen phosphate-dipotassium hydrogen phosphate.

In the above method, preferably, the Pinnick oxidation in the step (3) refers to an oxidation reaction carried out by using NaClO₂ and NaH₂PO₄, preferably, for example, in an acetonitrile-water solution, wherein the solvent used may be selected from any other suitable solvents. More preferably, the scavenger used may be one or more selected from the group consisting of resorcinol, aminosulfonic acid, 2-methyl-2-butene, dimethyl sulfoxide and hydrogen peroxide.

In the above method, preferably, the organic amine used in the step (3) may be one or more selected from the group consisting of methylamine, ethylamine, propylamine, cyclopropylamine, *n*-butylamine, *tert*-butylamine, *n*-pentylamine, isopentylamine, *tert*-pentylamine, cyclopentylamine, hexylamine, cyclohexylamine, diethylamine, ethylenediamine, diisopropylethylamine, triethylamine, ethanolamine, phenylamine, phenylethylamine and benzylamine.

In the above method, preferably, the halohydrocarbon R₁-X used in the step (4) may be selected from the group consisting of chlorobenzyl, bromobenzyl, chloro-C₁₋₆ alkyl, bromo-C₁₋₆ alkyl and iodo-C₁₋₆ alkyl.

In the above method, preferably, the base used in the step (4) may be one or more selected from the group consisting of sodium carbonate, potassium carbonate, cesium carbonate, sodium phosphate, potassium phosphate, triethylamine, diethylamine, methylamine, *tert*-butylamine and phenylamine.

In the above method, preferably, the acid used in the step (4) may be one or more selected from the group consisting of hydrochloric acid, hydrobromic acid, hydroiodic acid, sulfuric acid, nitric acid, phosphoric acid, formic acid, acetic acid and trifluoroacetic acid.

In the above method, preferably, the halogenated reagent containing halogen X used in the step (5) may be one or more selected from the group consisting of halogen, dichlorohydantoin, dibromohydantoin, diiodohydantoin, *N*-chlorosuccinimide (NCS), *N*-bromosuccinimide (NBS) and *N*-iodosuccinimide (NIS).

In the above method, preferably, the compound of Formula VI used may be prepared by reducing the compound of Formula X:

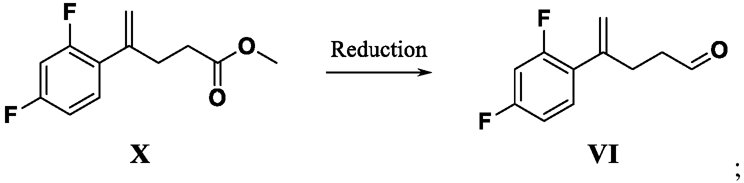

More preferably, the reducing agent used here may be one or more selected from the group consisting of lithium borohydride, sodium borohydride, potassium borohydride, sodium cyanoborohydride, potassium cyanoborohydride, borane, lithium aluminum hydride, diisobutylaluminum hydride and Red-Al.

Furthermore, the present disclosure also provides a method for preparing a compound of Formula IX as a key intermediate for posaconazole from a compound of Formula II, comprising the following step (1) and steps (6) to (8):
step (1): hydrolyzing a compound of Formula II in a mixed solvent of an organic solvent and water under an alkaline condition, and then subjecting the hydrolysate to an acid treatment to obtain the compound of Formula I;
step (6): reducing a compound of Formula I in an organic solvent in the presence of a reducing agent at a temperature in a range of -5~5°C to obtain a compound of Formula VII;
step (7): reacting the compound of Formula VII with 1,2,4-triazole in an organic solvent in the presence of a base and a phase transfer catalyst to obtain a compound of Formula VIII; and
step (8): reacting the compound of Formula VIII with p-toluenesulfonyl chloride in an organic solvent under an alkaline condition to obtain the compound of Formula IX, 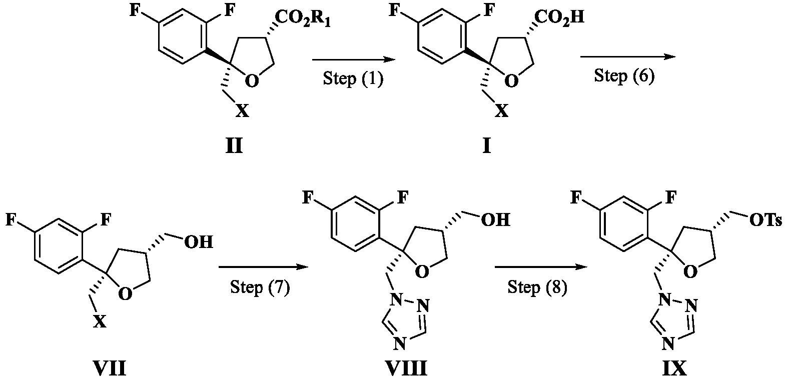 wherein X is Cl, Br or I; R₁ is an ester protection group; and Ts represents *p*-toluenesulfonyl.

More specifically, the step (1) is carried out completely same as the step (1) in the aforementioned method.

In the step (6), the reducing agent may be dissolved in the organic solvent, with the temperature controlled at -5~5°C, and then the compound of Formula I may be added, preferably a solution of the compound of Formula I dissolved in the same organic solvent may be added dropwise with the temperature maintained at -5~5°C until the reaction is completed. More preferably, in the step (6), a first reducing agent may be dissolved in the organic solvent with the temperature controlled at -5~5°C, and a solution of the compound of Formula I may be added dropwise for reaction while maintaining the temperature for 20-30 minutes. Thereafter, a second reducing agent is added dropwise at the same temperature with the temperature maintained at -5~5°C until the reaction is completed.

In the step (7), the base may be added to the organic solvent with the internal temperature controlled to be ≤ 40°C, then 1,2,4-triazole may be added in portions, and the mixture may be stirred for 2.0~2.5 hours. Then, the compound of Formula VII and the phase transfer catalyst are further added with the temperature raised to 95-100°C, and then the mixture is reacted while maintaining the temperature until the reaction is completed.

Preferably, the phase transfer catalyst used in the step (7) may be one or more selected from the group consisting of tetrabutylammonium iodide, tetrabutylammonium bromide and tetrabutylammonium fluoride.

In the step (8), the compound of Formula VIII is dissolved in the organic solvent, then the base and 4-dimethylaminopyridine (DMAP) are added with the temperature controlled at 5~10°C, and *p*-toluenesulfonyl chloride is added in portions. After that, the mixture is reacted at 15~20°C while maintaining the temperature until the reaction is completed.

In the above reaction, the organic solvent used in each of the steps may be one or more selected from the group consisting of methanol, ethanol, propanol, isopropanol, butanol, *tert*-butanol, 1,4-dioxane, *N-methylpyrrolidone, N,N-dimethylformamide, N*,*N*-dimethylacetamide, dimethyl sulfoxide, sulfolane, tetrahydrofuran, methyltetrahydrofuran, toluene, chlorobenzene, dichloromethane, trichloromethane, ethyl acetate, isopropyl acetate, butyl acetate, acetonitrile, diethyl ether, isopropyl ether, methyl *tert-butyl* ether, hexane, *n*-hexane, cyclohexane, and *n*-heptane.

Preferably, the organic solvent used in the step (6) may be one or more selected from the group consisting of methanol, ethanol, isopropanol, tetrahydrofuran, methyltetrahydrofuran, toluene, dichloromethane, ethyl acetate and isopropyl acetate.

Preferably, the organic solvent used in the step (7) may be one or more selected from the group consisting of 1,4-dioxane, dimethyl sulfoxide, *N*-methylpyrrolidone, *N,N*-dimethylformamide and *N*,*N*-dimethylacetamide.

Preferably, the organic solvent used in the step (8) may be one or more selected from the group consisting of dichloromethane, trichloromethane, diethyl ether, isopropyl ether, methyl *tert-butyl* ether, tetrahydrofuran, methyltetrahydrofuran, toluene, chlorobenzene, hexane, *n*-hexane, cyclohexane, n-heptane and acetonitrile.

In the above reaction, the reducing agent is one or more selected from the group consisting of lithium borohydride, sodium borohydride, potassium borohydride, sodium cyanoborohydride, potassium cyanoborohydride, borane, lithium aluminum hydride, diisobutylaluminum hydride, Red-Al, boron trifluoride diethyl etherate, aluminum chloride, ferric chloride and iodine.

Preferably, the reducing agent used in the step (6) (or the first and second reducing agents) may be one or more selected from the group consisting of lithium borohydride, sodium borohydride, potassium borohydride, sodium cyanoborohydride, potassium cyanoborohydride, borane, lithium aluminum hydride, diisobutylaluminum hydride and Red-Al. More preferably, the reducing agent used in the step (6) (or the first and second reducing agents) may be a combination of one or more of the above reducing agents with one or more selected from the group consisting of boron trifluoride diethyl etherate, aluminum chloride, ferric chloride and iodine.

In the above reaction, the base is selected from the group consisting of lithium carbonate, sodium carbonate, sodium bicarbonate, potassium carbonate, potassium bicarbonate, cesium carbonate, lithium hydroxide, sodium hydroxide, potassium hydroxide, sodium phosphate, potassium phosphate, disodium hydrogen phosphate, sodium dihydrogen phosphate, dipotassium hydrogen phosphate, potassium dihydrogen phosphate, sodium methoxide, sodium ethoxide, sodium *tert*-butoxide, potassium *tert*-butoxide, triethylamine, diethylamine, methylamine, *tert*-butylamine and phenylamine.

Preferably, the base used in the step (7) may be one or more selected from the group consisting of sodium methoxide, sodium ethoxide, sodium *tert*-butoxide and potassium *tert*-butoxide.

Furthermore, the present disclosure also provides some compounds as follows.

Compound of Formula II: 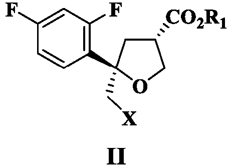 wherein X = Cl, Br or I; and R₁ is as defined above.

Specifically, the compound of Formula II may include the following compounds: 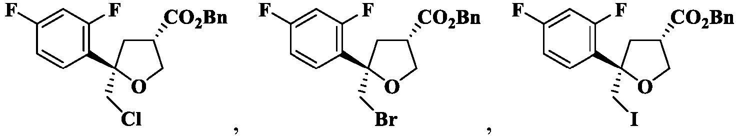 wherein Bn represents benzyl.

Other intermediate compounds, i.e., a compound of Formula III in which R₁ is benzyl, as well as a compound of Formula IV, a compound of Formula V and a compound of Formula VI:

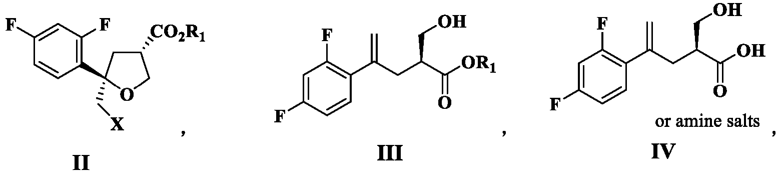

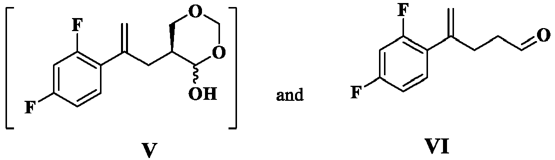

The method of the present disclosure will be further described below by way of examples. It should be appreciated that the following examples are provided merely for the purpose of better understanding the present disclosure, but not limiting the scope of the present disclosure in any way.

Unless stated otherwise, the raw materials and reagents used in the examples of the present disclosure are all commercially available or obtained by known methods, and are used as they are. The purity and chirality value are obtained through High Performance Liquid Chromatography (HPLC). The target products are identified through the consistency of the HPLC retention values with those of the standard products.

### Example 1: Preparation of the compound of Formula VI

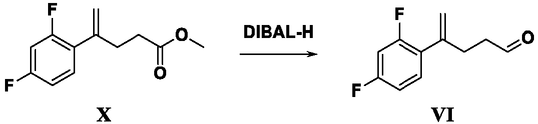

Under nitrogen protection, 40 g of the compound of Formula X and 200 g of toluene were added into a 500 mL reaction flask and stirred to be dissolved. The solution was cooled to a temperature of about -80°C with liquid nitrogen. 90 g of a 1.5 M solution of diisobutylaluminum hydride (DIBAL-H) in toluene was added dropwise. After that, the mixture was reacted for about 2.0 h while maintaining the temperature. Then, the reaction solution was controlled at a temperature in a range of -5~20°C, and was slowly added to 400 g of 3 M hydrochloric acid solution to quench the reaction. After that, the reaction solution was stirred for 3.0 h, and left to separate into layers. The organic layer was washed twice with water (80 g x 2) to obtain a solution of the compound of Formula VI in toluene. The solution obtained was directly used in the next reaction without concentration.

### Example 2: Preparation of the chiral catalyst (R-PDD-TMS)

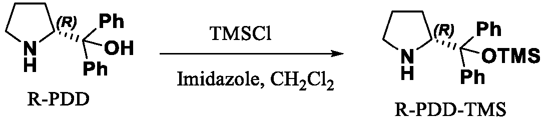

40 g of *R*-diphenylprolinol (R-PDD), 400 mL of dichloromethane and 32.2 g of imidazole were added into a 500 mL reaction flask and stirred to be dissolved. The solution was cooled to a temperature of 0°C. 50 mL of trimethylsilyl chloride (TMSCl) was added dropwise. After that, the mixture was warmed to a temperature of 25°C, and reacted for 12 h while maintaining the temperature. Thereafter, 1000 mL of methyl *tert-butyl* ether was added thereto. The mixture was stirred for 30 minutes while maintaining the temperature, and then filtered. The filtrate was washed with 500 mL water, then washed with 1000 mL saline, dried over anhydrous Na₂SO₄, and concentrated under reduced pressure to obtain 50 g oil. Yield: 97%, and HPLC purity: 98%.

### Example 3: Preparation of the compound of Formula V

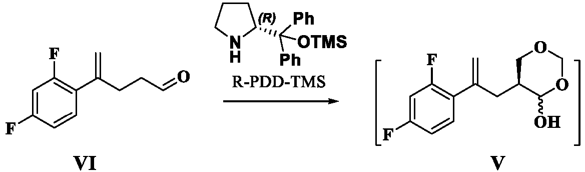

1.5 g of the chiral catalyst R-PDD-TMS, 2.0 g of KH₂PO₄ and 3.1 g of K₂HPO₄ were added to the solution of the compound of Formula VI prepared above in Example 1 in toluene, and stirred for 10~15 min. Then, 15 g of 37% formaldehyde solution was added. The mixture was warmed to an internal temperature of about 20°C, and reacted for 12 h while maintaining the temperature. 100 g water was added to the reaction solution and the mixture was stirred and separated into layers. The aqueous layer was extracted with 50 g toluene. Then, the aqueous layer was discarded, and the organic phases were combined, concentrated to dryness under reduced pressure to obtain 40 g of the compound of Formula V. Yield: 88%, and HPLC purity: 85%.

### Example 4: Preparation of the compound of Formula IV

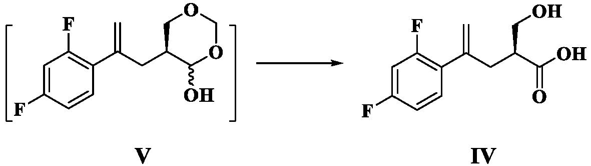

35 g of the compound of Formula V and 200 g of acetonitrile were added to a reaction flask, and stirred to dissolve. Thereafter, the solution was cooled to an internal temperature in a range of -5~5°C. 100 g of 30% hydrogen peroxide was added dropwise. After that, the temperature was controlled to be lower than 15°C. Then, 333 g of a NaClO₂/NaH₂PO₄/H₂O solution (40 g NaClO₂, 39 g NaH₂PO₄ and 254 g water) was added dropwise. Thereafter, the mixture was stirred for 1.0 h, warmed to room temperature, and reacted for 2.0 h while maintaining the temperature. The temperature was controlled to be ≤ 30°C. Then, 25 g of sodium hydrogen sulfite was added to quench the reaction. The reaction mixture was stirred for 1.5 h, and left to separate into layers. The aqueous layer was extracted with an appropriate amount of methyl *tert-butyl* ether, and the organic layers were combined and concentrated to dryness under reduced pressure. 100 g of water was added, and the pH was adjusted to 8.0~10.0 by adding liquid base. An appropriate amount of methyl *tert-butyl* ether was added. The mixture was stirred and left to separate into layers. The organic layer was discarded. The pH of the alkaline aqueous layer was adjusted to 2~4 by adding concentrated hydrochloric acid. 100 g of methyl *tert-butyl* ether was added. The mixture was stirred and left to separate into layers. The aqueous layer was discarded, and the organic layer was washed twice with water (60 g x 2), and concentrated to obtain 26.5 g of the compound of Formula IV as an oil. Yield: 80%, and HPLC purity: 85%.

### Example 5: Preparation of an amine salt of the compound of Formula IV

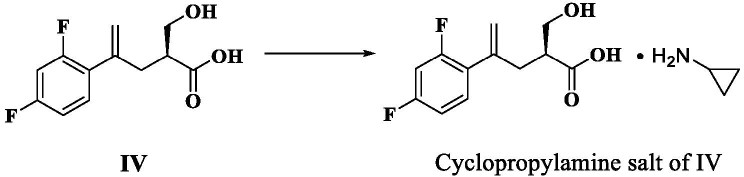

37 g of methyl *tert-butyl* ether was added to the compound of Formula IV obtained in Example 4. The mixture was warmed to a temperature in a range of 50~55°C, and stirred to be dissolved. 8.5 g (1.2 eq.) of cyclopropylamine was added dropwise. After that, the mixture was reacted for 1.0 h while maintaining the temperature, then cooled to a temperature in a range of 10-15°C, stirred for 2 h while maintaining the temperature, and filtered under suction to obtain 26.5 g of the cyclopropylamine salt of the compound of Formula IV. Yield: 81%, and HPLC purity: 95%.

### Example 6: Preparation of an amine salt of the compound of Formula IV

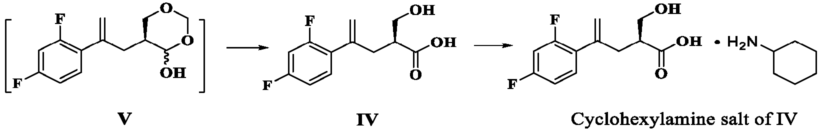

35 g of the compound of Formula V and 200 g of acetonitrile were added to a reaction flask, and stirred to be dissolved. Thereafter, the solution was cooled to an internal temperature in a range of -5~5°C. 100 g of 30% hydrogen peroxide was added dropwise. After that, the temperature was controlled to be lower than 15°C. Then, 333 g of a NaClO₂/NaH₂PO₄/H₂O solution (40 g NaClO₂, 39 g NaH₂PO₄ and 254 g water) was added dropwise. Thereafter, the mixture was stirred for 1.0 h, warmed to room temperature, and reacted for 2.0 h while maintaining the temperature. The temperature was controlled to be ≤ 30°C. Then, 25 g of sodium hydrogen sulfite was added to quench the reaction. The reaction mixture was stirred for 1.5 h, and left to separate into layers. The aqueous layer was extracted with an appropriate amount of methyl *tert-butyl* ether, and the organic layers were combined, and concentrated to dryness under reduced pressure. 100 g of water was added, and the pH was adjusted to 8.0~10.0 by adding liquid base. An appropriate amount of methyl *tert-butyl* ether was added. The mixture was stirred and left to separate into layers. The organic layer was discarded. The pH of the alkaline aqueous layer was adjusted to 2~4 by adding concentrated hydrochloric acid. 100 g of methyl *tert-butyl* ether was added. The mixture was stirred and left to separate into layers. The aqueous layer was discarded, and the organic layer was washed twice with water (60 g x 2). 14.8 g of cyclohexylamine was added dropwise to the organic phase under stirring. The mixture was stirred for 1 h, then cooled to 10~15°C, stirred for 2 h while maintaining the temperature, and filtered under suction to obtain 39.6 g of the cyclohexylamine salt of the compound of Formula IV.

### Example 7: Preparation of the compound of Formula III (R₁ is benzyl (Bn))

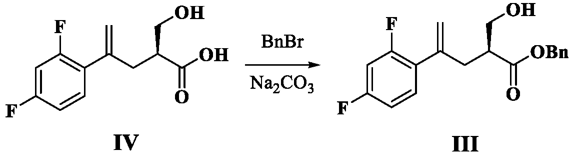

35 g of *N,N*-dimethylformamide and 8.48 g of the compound of Formula IV were added into a reaction flask, and stirred to be dissolved. Then, 4.5 g of sodium carbonate and 6.0 g of benzyl bromide were added. The reaction mixture was controlled at a temperature in a range of 5~20°C, and reacted for 3.0 h while maintaining the temperature. After that, 120 g of methyl *tert-butyl* ether was added, and the mixture was stirred for about 10~15 min and filtered. The filtrate was washed three times with water (60 g x 3), and separated into layers. The aqueous layer was discarded, and the organic layer was concentrated to dryness to obtain 10.5 g of the compound of Formula III. Yield: 90%, and HPLC purity: 90%.

### Example 8: Preparation of the compound of Formula III (R₁ is ethyl (Et))

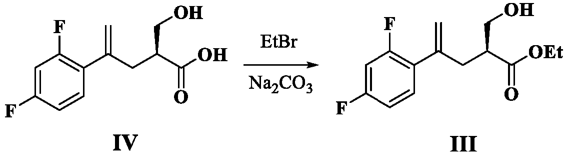

70 g of *N*,*N*-dimethylformamide and 17 g of the compound of Formula IV were added into a reaction flask, and stirred to be dissolved. Then, 9 g of sodium carbonate and 7.8 g of ethyl bromide were added. The reaction mixture was controlled at a temperature in a range of 5~20°C, and reacted for 3.0 h while maintaining the temperature. After that, 240 g of methyl *tert-butyl* ether was added, and the mixture was stirred for about 30 min and filtered. The filtrate was washed three times with water (120 g x 3), and separated into layers. The aqueous layer was discarded, and the organic layer was concentrated to dryness to obtain 16.5 g of the compound of Formula III. Yield: 87%.

### Example 9: Preparation of the compound of Formula III (R₁ is methyl (Me))

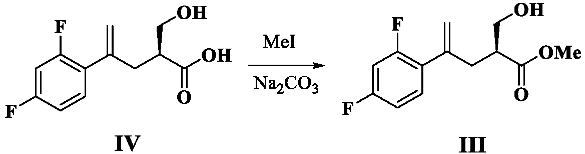

75 g of *N,N*-dimethylformamide and 20 g of the compound of Formula IV were added into a reaction flask, and stirred to be dissolved. Then, 10.5 g of sodium carbonate and 11.95 g of iodomethane were added. The reaction mixture was controlled at a temperature in a range of 5~20°C, and reacted for 3.0 h while maintaining the temperature. After that, 240 g of methyl *tert-butyl* ether was added, and the mixture was stirred for about 30 min and filtered. The filtrate was washed three times with water (120 g x 3), and separated into layers. The aqueous layer was discarded, and the organic layer was concentrated to dryness to obtain 19.4 g of the compound of Formula III. Yield: 91.5%.

### Example 10: Preparation of the compound of Formula III (R₁ is benzyl (Bn))

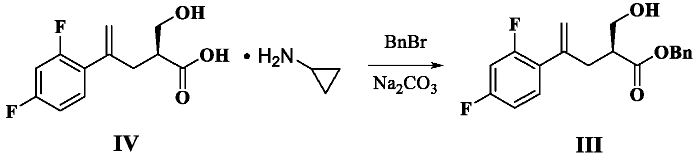

10.48 g of the cyclopropylamine salt of the compound of Formula IV and 50 g of water were added into a reaction flask, and uniformly stirred. Then, concentrated hydrochloric acid was added to adjust the pH to 1~3, and 50 g of methyl *tert-butyl* ether was added. The mixture was left to separate into layers. The aqueous layer was extracted with 20 g of methyl *tert-butyl* ether, and the organic layers was combined and washed with water (50 g x 2). The aqueous layer was discarded, and the organic layer was concentrated to dryness under reduced pressure. 35 g of *N,N*-dimethylformamide was added to the concentrate, and stirred to be dissolved. Then, 4.5 g of sodium carbonate and 6.0 g of benzyl bromide were added. The reaction mixture was controlled at a temperature in a range of 5~20°C, and reacted for 3.0 h while maintaining the temperature. After that, 120 g of methyl *tert-butyl* ether was added, and the mixture was stirred for about 10-15 min and filtered. The filtrate was washed three times with water (60 g x 3), and separated into layers. The aqueous layer was discarded, and the organic layer was concentrated to dryness to obtain 10.5 g of the compound of Formula III. Yield: 90.3%, and HPLC purity: 95.5%.

### Example 11: Preparation of the compound of Formula II (in which R₁ is benzyl (Bn) and X is Br)

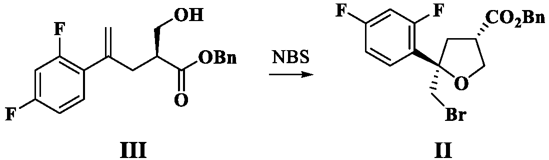

68.6 g of the compound of Formula III, 1170 g of tetrahydrofuran and 50 g of water were added into a reaction flask, and stirred to be dissolved. The temperature was controlled in a range of -5~5°C, and 41.2 g of N-bromosuccinimide (NBS) was added in portions. The mixture was reacted for 3.0 h while maintaining the temperature. After that, 500 g of 10% NaHSO₃ was added to the reaction solution. The mixture was stirred for 30 min, and then concentrated to dryness under reduced pressure. 210 g of methyl *tert-butyl* ether was added. The mixture was washed with water (100 g x 2). The aqueous layer was discarded, and the organic layer was concentrated to dryness to obtain 68 g of the compound of Formula II. Yield: 80.1%, and HPLC purity: 90.5%.

### Example 12: Preparation of the compound of Formula II (in which R₁ is ethyl (Et) and X is Br)

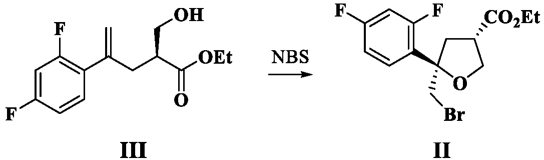

56.8 g of the compound of Formula III, 1170 g of tetrahydrofuran and 50 g of water were added into a reaction flask, and stirred to be dissolved. The temperature was controlled in a range of -5~5°C, and 41.2 g of N-bromosuccinimide (NBS) was added in portions. The mixture was reacted for 3.0 h while maintaining the temperature. After that, 500 g of 10% NaHSO₃ was added to the reaction solution. The mixture was stirred for 30 min, and then concentrated to dryness under reduced pressure. 210 g of methyl *tert-butyl* ether was added. The mixture was washed with water (100 g x 2). The aqueous layer was discarded, and the organic layer was concentrated to dryness to obtain 60.8 g of the compound of Formula II. Yield: 83%.

### Example 13: Preparation of the compound of Formula II (R₁ is methyl (Me) and X is Br)

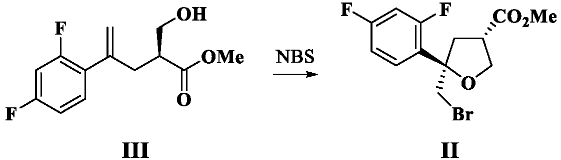

53.1 g of the compound of Formula III, 1170 g of tetrahydrofuran and 50 g of water were added into a reaction flask, and stirred to be dissolved. The temperature was controlled in a range of -5~5°C, and 41.2 g of N-bromosuccinimide (NBS) was added in portions. The mixture was reacted for 3.0 h while maintaining the temperature. After that, 500 g of 10% NaHSO₃ was added to the reaction solution. The mixture was stirred for 30 min, and then concentrated to dryness under reduced pressure. 210 g of methyl *tert-butyl* ether was added. The mixture was washed with water (100 g x 2). The aqueous layer was discarded, and the organic layer was concentrated to dryness to obtain 56.7 g of the compound of Formula II. Yield: 81.8%.

### Example 14: Preparation of the compound of Formula I (X is Br)

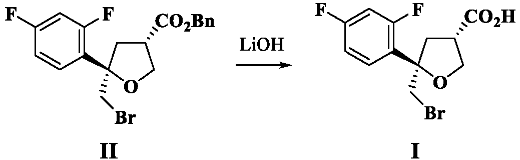

73 g of the compound of Formula II, 170 g of tetrahydrofuran and 13.2 g of water were added into a reaction flask. The mixture was cooled to a temperature in a range of 0~5°C. 65.7 mL of 20% LiOH solution was added dropwise. Thereafter, the mixture was stirred for 0.5 h, and reacted at about 2°C for 7 h. The reaction mixture was extracted with 146 g of ethyl acetate, and left to separate into layers. The aqueous layer was extracted with ethyl acetate (73 g x 2), and the ethyl acetate phase was discarded. Concentrated hydrochloric acid was added to the aqueous layer to adjust the pH to 2~3. Then, 73 g of ethyl acetate was added, and the mixture was stirred and left to separate into layers. The aqueous layer was back extracted with ethyl acetate (73 g x 2), and the ethyl acetate layers were combined and further washed with 146 g of water. The organic layer was concentrated under reduced pressure to obtain 50 g of the compound of Formula I as an oil. Yield: 87.7%, and HPLC purity: 91%.

### Example 15: Preparation of the compound of Formula I (X is Br)

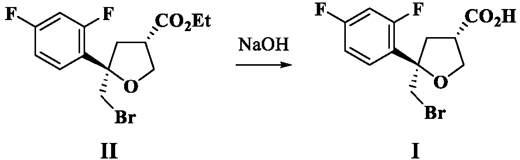

62 g of the compound of Formula II, 170 g of tetrahydrofuran and 13.2 g of water were added into a reaction flask. The mixture was cooled to a temperature in a range of 0~5°C. 73 g of 30% NaOH solution was added dropwise. Thereafter, the mixture was stirred for 0.5 h, and reacted at about 3°C for 5 h. The reaction mixture was extracted with 150 g of ethyl acetate, and left to separate into layers. The aqueous layer was extracted with ethyl acetate (50 g x 2), and the ethyl acetate phase was discarded. Concentrated hydrochloric acid was added to the aqueous layer to adjust the pH to 2~3. Then, 50 g of ethyl acetate was added, and the mixture was stirred and left to separate into layers. The aqueous layer was back extracted with ethyl acetate (50 g x 2), and the ethyl acetate layers were combined and further washed with 150 g of water. The organic layer was concentrated under reduced pressure to obtain 51.3 g of the compound of Formula I as an oil. Yield: 92%, and HPLC purity: 90%.

### Example 16: Preparation of the compound of Formula I (X is Br)

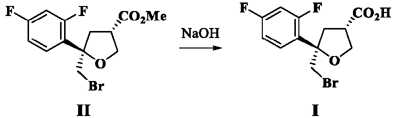

59.5 g of the compound of Formula II, 170 g of tetrahydrofuran and 13.2 g of water were added into a reaction flask. The mixture was cooled to a temperature in a range of 0~5°C. 73 g of 30% NaOH solution was added dropwise. Thereafter, the mixture was stirred for 0.5 h, and reacted at about 3°C for 5 h. The reaction mixture was extracted with 150 g of ethyl acetate, and left to separate into layers. The aqueous layer was extracted with ethyl acetate (50 g x 2), and the ethyl acetate phase was discarded. Concentrated hydrochloric acid was added to the aqueous layer to adjust the pH to 2~3. Then, 50 g of ethyl acetate was added, and the mixture was stirred and left to separate into layers. The aqueous layer was back extracted with ethyl acetate (50 g x 2), and the ethyl acetate layers were combined and further washed with 150 g of water. The organic layer was concentrated under reduced pressure to obtain 50.7 g of the compound of Formula I as an oil. Yield: 89%, and HPLC purity: 95.5%.

### Example 17: Preparation of a cyclohexylamine salt the compound of Formula I (X is Br)

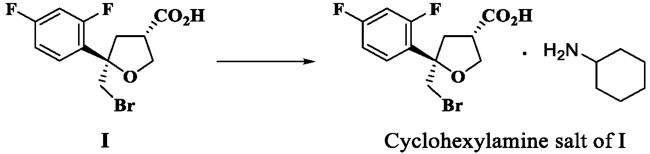

75 g of methyl *tert-butyl* ether was added to the compound of Formula I obtained in Example 14. The mixture was warmed to a temperature in a range of 50~55°C, and stirred to be dissolved. 15.4 g of cyclohexylamine was added dropwise. After that, the mixture was reacted for 1.0 h while maintaining the temperature, then cooled to a temperature approximately in a range of 10-15°C, stirred for 3 h while maintaining the temperature, filtered under suction, and dried to obtain 55 g of the cyclohexylamine salt of the compound of Formula I. Yield: 84.6%, HPLC purity: 97%, and ee value determined by normal phase HPLC: 98%.

### Example 18: Preparation of a cyclopropylamine salt the compound of Formula I (X is Br)

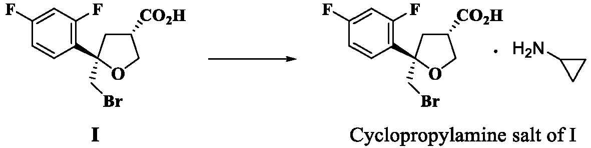

80 g of methyl *tert-butyl* ether was added to the compound of Formula I obtained in Example 15. The mixture was warmed to a temperature in a range of 50~55°C, and stirred to be dissolved. 9.12 g of cyclopropylamine was added dropwise. After that, the mixture was reacted for 1.0 h while maintaining the temperature, then cooled to a temperature approximately in a range of 10~15°C, stirred for 3 h while maintaining the temperature, filtered under suction, and dried to obtain 52 g of the cyclopropylamine salt of the compound of Formula I. Yield: 86%, HPLC purity: 96.5%, and ee value determined by normal phase HPLC: 98%.

### Example 19: Preparation of the compound of Formula IX as a key intermediate for posaconazole from the compound of Formula I (X is Br)

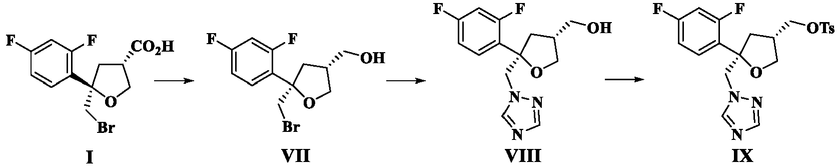

### (1) Preparation of Compound VII:

Under nitrogen protection, 100 g of THF and 14.4 g of NaBH₄ were added into a 1 L reaction flask. The reaction temperature was controlled to be in a range of -5~5°C. 350 g of THF solution containing 116 g of the compound of Formula I was added dropwise. Thereafter, the internal temperature was controlled to be in a range of -5~5°C, and the temperature was maintained for 30 min. The internal temperature was controlled to be in a range of -5~5°C, and 42.6 g of boron trifluoride diethyl etherate was added dropwise over 1~2 h. After that, the internal temperature was controlled to be in a range of -5~5°C, and the temperature was maintained for about 2 h. After the reaction was completed, the reaction solution was slowly added to 400 g of 10% HCl solution (40 g HCl + 360 g drinking water) which was precooled to 0~5°C. The mixture was controlled at a temperature in a range of 10~25°C, stirred for 30 min, and separated into phases. The THF layer was set aside, and the aqueous layer was extracted wit toluene (100 g x 2), and separated into layers. The aqueous layer was discarded. The organic layers were combined, washed with 200 g of saturated NaHCO₃ solution, and separated into layers. The organic layer was washed with 200 g of water, and the aqueous layer was discarded. The organic phase was concentrated to dryness under reduced pressure with the external temperature controlled to be ≤ 60°C to obtain 108.7 g of the compound of Formula VII. Yield: 98%, and HPLC purity>95%.

### (2) Preparation of Compound VIII:

Under nitrogen protection, 264 g of DMSO and 121.7 g of potassium *tert*-butoxide were added into a dry and clean reaction flask. After that, the internal temperature was controlled to be ≤ 40°C, and 77.0 g of 1,2,4-triazole was added in portions. Thereafter, the internal temperature was controlled to be in a range of 20~40°C, and the mixture was reacted for 2 h while maintaining the temperature. After that, 118 g of the compound of Formula VII, 8.6 g tetrabutylammonium iodide, and 7.3 g of 18-crown-6 were added. The mixture was slowly warmed to a temperature in a range of 95~100°C with the internal temperature controlled in a range of 95~100°C, and reacted for 36 h while maintaining the temperature. Thereafter, the mixture was cooled to a temperature below 30°C, and was added to 600 g of drinking water to quench the reaction. The material was extracted with 600 g of methyl *tert-butyl* ether. The methyl *tert-butyl* ether layer was washed with drinking water, supplemented with 30 g of anhydrous Na₂SO₄ and 4 g of activated carbon, dried and decolorized for 1 h, and filtered under suction. The filter cake was leached with methyl *tert-butyl* ether (30 g x 2). The filtrates were combined, with an external temperature controlled to be ≤ 60°C, and concentrated to dryness to obtain 85 g of the compound of Formula VIII. Yield: 75%, and HPLC purity>96.7%.

### (3) Preparation of Compound IX:

Under nitrogen protection, 240 g of DCM, 1.5 g of DMAP, 35.5 g of the compound of Formula VIII and 30.8 g of triethylamine were added into a dry and clean reaction flask. The mixture was cooled to a temperature in a range of 5~10°C with the internal temperature controlled to be in a range of 5~10°C, and 23.3 g of p-toluenesulfonyl chloride was added in portions. Thereafter, the mixture was reacted with the internal temperature controlled to be in a range of 15-20°C. After the reaction was completed, the reaction mixture was added to 300 g of water to quench the reaction, and separated into layers. The DCM layer was washed sequentially with a Na₂CO₃ solution, a 10% HCl solution and water, and separated into layers. Then, the aqueous layer was discarded, and the DCM layer was concentrated to dryness under reduced pressure with the external temperature controlled to be ≤ 60°C. 300 g of isopropanol was added. The mixture was warmed to a temperature of 50°C, stirred to be dissolved, and slowly cooled to a temperature in a range of 0~5°C to crystallize for 2 h. The reaction mixture was filtered under suction to obtain 48.5 g of the compound of Formula IX. Yield: 89.8%, HPLC purity: 99.0%, and ee value: 99.5%.

The above particular embodiments and examples are used for describing the objects, technical solutions and advantageous effects of the present disclosure in detail. It should be understood that the above particular embodiments and examples are only for the purpose of illustrating the present disclosure, but not intended to limit the scope of the present disclosure. Any modification, equivalent replacement, improvement and the like made within the spirit and principle of the present disclosure should be included within the protection scope of the present disclosure.

## Claims

1. A method for preparing a compound of Formula I as an intermediate for posaconazole, 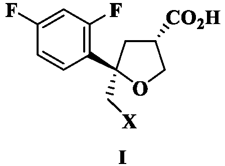
the method comprises the following step (1): hydrolyzing a compound of Formula II in a mixed solvent of an organic solvent and water under an alkaline condition, and then subjecting the hydrolysate to an acid treatment to obtain the compound of Formula I, 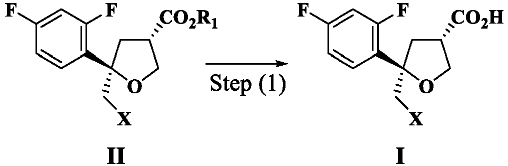
wherein X is Cl, Br or I, and R₁ is an ester protection group.

2. The method according to claim 1, wherein R₁ is selected from the group consisting of C₁₋₈ alkyl, C₃₋₈ cycloalkyl, C₆₋₁₀ aryl, C₁₋₈ alkyl-substituted C₆₋₁₀ aryl, benzyl, C₁₋₈ alkyl-substituted benzyl, C₁₋₈ alkoxy-substituted benzyl and halogenated benzyl; preferably, R₁ is selected from the group consisting of C₁₋₈ alkyl, benzyl, C₁₋₈ alkyl-substituted benzyl, C₁₋₈ alkoxy-substituted benzyl and halogenated benzyl.

3. The method according to claim 1, wherein the hydrolysis in the step (1) is carried out in the presence of one or more bases selected from the group consisting of lithium hydroxide, sodium hydroxide, potassium hydroxide, sodium carbonate, potassium carbonate, sodium bicarbonate and potassium bicarbonate.

4. The method according to claim 1, wherein the acid treatment comprises adjusting a pH of an aqueous phase obtained after the reaction to 2~3 with one or more organic acids or inorganic acids; preferably, an organic amine is added to the solution obtained after the acid treatment to obtain an amine salt of the compound of Formula I.

5. The method according to claim 1, wherein the compound of Formula II is prepared by a method comprising the following steps (2) to (5):
step (2): reacting a compound of Formula VI with formaldehyde or paraformaldehyde in an organic solvent in the presence of a chiral catalyst to obtain a compound of Formula V;
step (3): subjecting the compound of Formula V to a Pinnick oxidation using NaClO₂ and NaH₂PO₄ in the presence of a scavenger to obtain a compound of Formula IV;
step (4): reacting the compound of Formula IV with a halohydrocarbon R₁-X under an alkaline condition to obtain a compound of Formula III; and
step (5): subjecting the compound of Formula III to a halogenation and cyclization reaction in the presence of a halogenated reagent containing halogen X to obtain the compound of Formula II,
or
step (2): reacting a compound of Formula VI with formaldehyde or paraformaldehyde in an organic solvent in the presence of a chiral catalyst to obtain a compound of Formula V;
step (3): subjecting the compound of Formula V to a Pinnick oxidation using NaClO₂ and NaH₂PO₄ in the presence of a scavenger, and adding an organic amine compound to obtain an organic amine salt of the compound of Formula IV;
step (4): reacting the organic amine salt of the compound of Formula IV with a halohydrocarbon R₁-X under an alkaline condition to obtain a compound of Formula III; and
step (5): subjecting the compound of Formula III to a halogenation and cyclization reaction in the presence of a halogenated reagent containing halogen X to obtain the compound of Formula II, 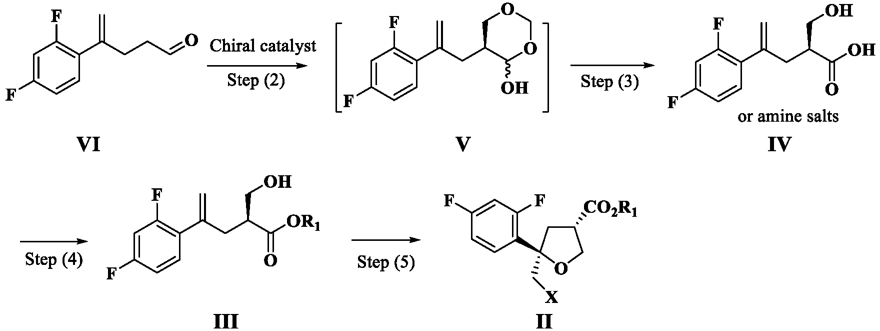 wherein X and R₁ are as defined above.

6. The method according to claim 5, wherein the chiral catalyst used in the step (2) is a compound of the following formula: 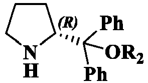 wherein R₂ is selected from the group consisting of trimethylsilyl, triethylsilyl, *tert*-butyldimethylsilyl, triisopropylsilyl, benzyl, 4-methylbenzyl and phenyl, and Ph represents phenyl.

7. The method according to claim 5, wherein the Pinnick oxidation is performed in an acetonitrile-water solution in the presence of NaClO₂ and NaH₂PO₄; preferably, the scavenger is one or more selected from the group consisting of resorcinol, aminosulfonic acid, 2-methyl-2-butene, dimethyl sulfoxide and hydrogen peroxide.

8. The method according to claim 5, wherein the halogenated reagent containing halogen X used in the step (5) is one or more selected from the group consisting of halogen, dichlorohydantoin, dibromohydantoin, diiodohydantoin, *N*-chlorosuccinimide (NCS), *N*-bromosuccinimide (NBS) and *N*-iodosuccinimide (NIS); preferably, the organic amine is one or more selected from the group consisting of methylamine, ethylamine, propylamine, cyclopropylamine, *n*-butylamine, *tert*-butylamine, *n*-pentylamine, isopentylamine, *tert*-pentylamine, cyclopentylamine, hexylamine, cyclohexylamine, diethylamine, ethylenediamine, diisopropylethylamine, triethylamine, ethanolamine, phenylamine, phenylethylamine and benzylamine.

9. The method according to claim 5, wherein the compound of Formula VI is prepared by reducing a compound of Formula X: 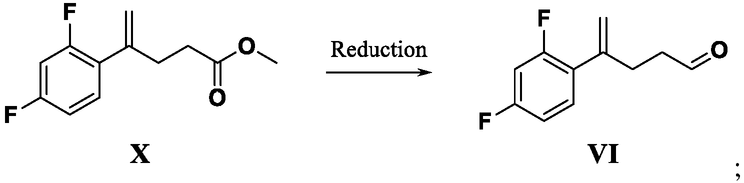 preferably, the reducing agent used for the reduction is one or more selected from the group consisting of lithium borohydride, sodium borohydride, potassium borohydride, sodium cyanoborohydride, potassium cyanoborohydride, borane, lithium aluminum hydride, diisobutylaluminum hydride and Red-Al.

10. A method for preparing a compound of Formula IX as a key intermediate for posaconazole from a compound of Formula II, comprising the following step (1) and steps (6) to (8):
step (1): hydrolyzing a compound of Formula II in a mixed solvent of an organic solvent and water under an alkaline condition, and then subjecting the hydrolysate to an acid treatment to obtain the compound of Formula I;
step (6): reducing the compound of Formula I in an organic solvent in the presence of a reducing agent at a temperature in a range of -5~5°C to obtain a compound of Formula VII;
step (7): reacting the compound of Formula VII with 1,2,4-triazole in an organic solvent in the presence of a base and a phase transfer catalyst to obtain a compound of Formula VIII; and
step (8): reacting the compound of Formula VIII with *p*-toluenesulfonyl chloride in an organic solvent under an alkaline condition to obtain the compound of Formula IX, 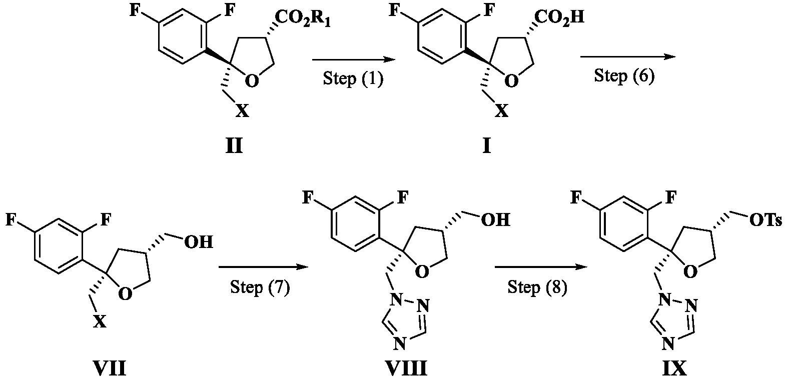 wherein X is Cl, Br or I; R₁ is an ester protection group; and Ts represents *p*-toluenesulfonyl.

11. The method according to claim 10, wherein the reducing agent used in the step (6) is one or more selected from the group consisting of lithium borohydride, sodium borohydride, potassium borohydride, sodium cyanoborohydride, potassium cyanoborohydride, borane, lithium aluminum hydride, diisobutylaluminum hydride and Red-Al; preferably, the reducing agent is a combination of one or more selected from the group consisting of lithium borohydride, sodium borohydride, potassium borohydride, sodium cyanoborohydride, potassium cyanoborohydride, borane, lithium aluminum hydride, diisobutylaluminum hydride and Red-Al with one or more selected from the group consisting of boron trifluoride diethyl etherate, aluminum chloride, ferric chloride and iodine.

12. The method according to claim 10, wherein the compound of Formula II is prepared by the method of claim 5; preferably, the compound of Formula II is prepared by the method of claim 9.

13. A compound of Formula II prepared by the method of claim 5: 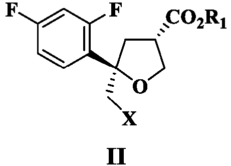 wherein X and R₁ are as defined above.

14. Compounds of Formula II, Formula III, Formula IV, Formula V and Formula VI prepared by the method of claim 9: 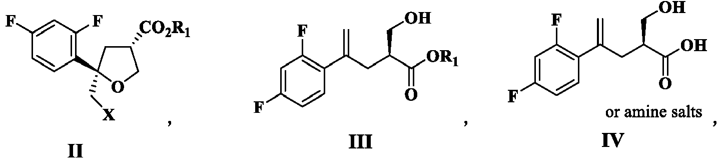 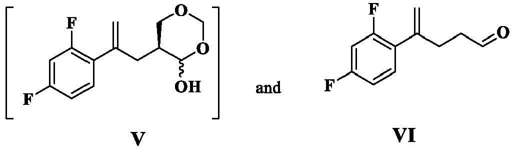 wherein R₁ is benzyl, and X is Cl, Br or I.
